Europäisches Patentamt

European Patent Office

Office européen des brevets

(11) Numéro de publication : **0 298 839 B1**

(12) ## FASCICULE DE BREVET EUROPEEN

(45) Date de publication du fascicule du brevet :
**04.03.92 Bulletin 92/10**

(51) Int. Cl.⁵ : **A61K 7/16**

(21) Numéro de dépôt : **88401693.2**

(22) Date de dépôt : **30.06.88**

(54) **Dentifrice à indicateur de temps de brossage.**

(30) Priorité : **06.07.87 FR 8709551**

(43) Date de publication de la demande :
**11.01.89 Bulletin 89/02**

(45) Mention de la délivrance du brevet :
**04.03.92 Bulletin 92/10**

(84) Etats contractants désignés :
**AT BE CH DE ES GB GR IT LI LU NL SE**

(56) Documents cités :
**EP-A- 0 218 732**
**CH-A- 215 518**
**CHEMICAL ABSTRACTS, vol. 82, no. 8, 21 avril 1975, page 349, résumé no. 103032j, Columbus, Ohio, US; & JP-A-74 71 151 (LION DENTIFRICE CO., LTD) 10-07-1974**

(56) Documents cités :
**CHEMICAL ABSTRACTS, vol. 107, no. 18, 2 novembre 1987, résumé no. 161424j, Columbus, Ohio, US; & JP-A-62 89 613 (KAO CORP.) 24-04-1987**
**CHEMICAL ABSTRACTS, vol. 104, no. 4, janvier 1986, page 290, résumé no. 24079z, Columbus, Ohio, US; && JP-A-60 40 401 (LION CORP.) 11-09-1985**

(73) Titulaire : **Romeo, Roch**
**9, rue Treilhard**
**F-75008 Paris (FR)**

(72) Inventeur : **Romeo, Roch**
**9, rue Treilhard**
**F-75008 Paris (FR)**

(74) Mandataire : **Armengaud Ainé, Alain**
**Cabinet ARMENGAUD AINE 3 Avenue Bugeaud**
**F-75116 Paris (FR)**

## Description

La présente invention concerne des perfectionnements apportés aux dentifrices.

Il est bien connu que, dans l'hygiène dentaire, le temps de brossage des dents est un facteur particulièrement important. Ce temps de brossage est généralement difficile à apprécier et, jusqu'à présent, aucune solution efficace et pratique n'a été apportée pour résoudre ce problème. On a bien tenté (Chemical Abstracts vol 82 N° 8, 21 Avril 1975 page 349, résumé N° 103032j) de résoudre ce problème en incorporant à la pâte dentifrice un piquent naturel absorbé par des absorbants tels que la cellulose, afin d'obtenir une indication relative à un nettoyage efficace des dents, par une modification de la couleur. On retrouve la même préoccupation dans "Chemical Abstracts vol. 104 N° 4, Janvier 1986, page 290, résumé N° 24079z qui décrit une composition de pâte dentifrice à variation de couleur lors de l'application qui comprend un colorant et un agent réducteur.

De même EP-A-0218 732 décrit une composition de dentifrice qui comporte au moins un indicateur de couleur dont la couleur se modifie dans le dentifrice, lors de son utilisation, après une période de temps prédéterminée de brossage.

On connaît par ailleurs par CH-A-215 218 l'utilisation d'une matière colorante à savoir le bleu de méthylène ou un produit de réduction d'une telle matière permettant d'obtenir des propriétés antiseptiques améliorées afin de donner aux dents une blancheur plus parfaite.

L'expérience révèle qu'aucune des techniques antérieures ne permet, d'une part, d'obtenir une bonne coloration initiale de la pâte dentifrice et, d'autre part, une décoloration franche du dentifrice après un temps de brossage efficace, généralement évalué à 3 minutes environ. La présente invention se propose d'apporter une nouvelle composition de dentifrice permettant à l'utilisateur de connaître, avec une précision suffisante, le temps de brossage minimal nécessaire à un brossage efficace.

En conséquence, cette invention a pour objet un dentifrice comportant une addition d'un colorant et d'un agent réducteur pour décolorer ledit dentifrice sous l'effet du brossage, caractérisé en ce qu'il est constitué d'un dentifrice à base de fluor, du type disponible dans le commerce, comportant du bleu de méthylène en une proportion de l'ordre de 500 µl à 1 ml, en solution à 1 mg/ml, pour 5 g de dentifrice, soit une proportion de l'ordre de 0,125 à 0,250 µM par gramme de dentifrice et un agent réducteur constitué par de l'ascorbate de sodium sous forme pulvérulente qui est ajouté au moment de l'emploi.

Selon une caractéristique de la présente invention, l'agent réducteur est ajouté au dentifrice coloré selon une proportion de l'ordre de 90 mg pour 5 g de dentifrice.

On a donné ci-après un exemple de réalisation d'un dentifrice perfectionné selon la présente invention.

On a préparé une solution aqueuse de bleu de méthylène à 1mg/ml. A 5g de dentifrice à base de fluor, actuellement disponible dans le commerce, pesé dans une cupule de porcelaine ou de pyrex, on a mélangé 500 µl ou 1ml de la solution de bleu de éthylène, soit 0,125, ou 0,250µM par gramme de dentifrice. La concentration est choisie de façon à obtenir une bonne coloration initiale du dentifrice.

Au dentifrice coloré, on a ajouté, au moment de l'emploi, un agent réducteur constitué par de l'ascorbate de sodium pulvérulent, selon une proportion de 90mg d'ascorbate de sodium pour 5g de dentifrice. On a observé une décoloration franche du dentifrice après un temps de brossage de 3 minutes environ.

En vue d'une mise en oeuvre pratique du dentifrice objet de la présente invention, celui-ci est conditionné dans un tube classique comportant une ou plusieurs colonnes dans lesquelles est placé l'ascorbate de sodium, le dentifrice classique à base de fluor occupant le volume cylindrique disponible autour des colonnes d'ascorbate de sodium, le mélange dentifrice + ascorbate de sodium étant ainsi réalisé au moment de l'emploi par pressage sur le tube de façon usuelle.

Il demeure bien entendu que la présente invention n'est pas limitée à l'exemple de réalisation décrit ci-dessus mais qu'elle en englobe toutes les variantes.

## Revendications

1. Dentifrice comportant une addition d'un colorant et d'un agent réducteur pour décolorer ledit dentifrice sous l'effet du brossage, caractérisé en ce qu'il est constitué d'un dentifrice à base de fluor, du type disponible dans le commerce, comportant du bleu de méthylène en une proportion de l'ordre de 500 µl à 1 ml, en solution à 1 mg/ml, pour 5 g de dentifrice, soit une proportion de l'ordre de 0,125 à 0,250 µM par gramme de dentifrice et un agent réducteur constitué par de l'ascorbate de sodium sous forme pulvérulente qui est ajouté au moment de l'emploi.

2. Dentifrice selon la revendication 1, caractérisé en ce que l'agent réducteur est ajouté au dentifrice coloré selon une proportion de l'ordre de 90 mg pour 5 g de dentifrice.

3. Dentifrice selon la revendication 1, caractérisé en ce qu'il est conditionné dans un tube classique comportant une ou plusieurs colonnes renfermant l'agent réducteur, constitué de préférence d'ascorbate de sodium, la partie restante du tube contenant le dentifrice classique préalablement coloré par le bleu de méthylène, le mélange du dentifrice coloré et de l'agent réducteur s'effectuant au moment de l'emploi par pressage sur le tube de façon classique.

of use by pressing the tube in the standard manner.

## Patentansprüche

1. Zahnpasta, die einen Zusatz eines Farbstoffes und eines Reduktionsmiffels zum Entfärben dieser Zahnpaste beim Zähnebürsten aufweist, dadurch **gekennzeichnet,** daß sie aus einer im Handel erhältlichen Zahnpasta auf Fluorbasis besteht, die Methylenblau in einem Anteil von 500 μl bis 1 ml, als Lösung mit 1 mg/ml, pro 5 g Zahnpasta, wobei dies einem Anteil von 0,125 bis 0,250 μM pro Gramm Zahnpasta entspricht, und ein Reduktionsmittel enthält, das aus pulverförmigen Natriumascorbat besteht, welches zum Anwendungszeifpunkt hinzugegeben wird.

2. Zahnpasta nach Anspruch 1, dadurch **gekennzeichnet,** daß das Reduktionsmittel in einem Anteil von 90 mg pro 5 g Zahnpasta zur gefärbten Zahnpasfa hinzugegeben wird.

3. Zahnpasfa nach Anspruch 1, dadurch **gekennzeichnet,** daß sie in einer klassischen Tube konditioniert ist, die eine oder mehrere Säulen besitzt, die das vorzugsweise aus Natriumascorbat bestehende Reduktionsmittel enthalten, wobei der restliche Teil der Tube die klassische, zuvor mit Methylenblau gefärbte Zahnpasta enthält und wobei die Vermischung der gefärbten Zahnpasfa und des Reduktionsmittels zum Anwendungszeitpunkt durch Drücken auf die Tube in klassischer Weise bewirkt wird.

## Claims

1. A tooth-paste comprising an addition of a colouring agent and of a reducing agent for discolouring said tooth-paste under the effect of the brushing, characterized in that it is made of a fluorine-based tooth-paste, of the type available in the trade, including methylene blue in a proportion of the order of 500 μl to 1 ml, in a 1 mg/ml solution, for 5 g of tooth-paste, that is a proportion of the order of 0.125 to 0.250 μM per gram of tooth-paste, and a reducing agent constituted by sodium ascorbate in powder form which is added at the moment of use.

2. A tooth-paste according to claim 1, characterized in that a reducing agent is added to the coloured tooth-paste in a proportion of the order of 90 mg for 5 g of tooth-paste.

3. A tooth-paste according to claim 1, characterized in that it is conditioned in a standard tube comprising one or several columns in which is placed the reducing agent, constituted preferably of sodium ascorbate, the remaining portion of the tube containing the standard tooth-paste, previously coloured with methylene blue, the coloured tooth-paste plus reducing agent mixture being thus provided at the moment